# EUROPEAN PATENT SPECIFICATION

(11) **EP 2 747 740 B1**
(45) Date of publication and mention of the grant of the patent: **07.08.2019**
(21) Application number: 12769413.1
(22) Date of filing: 03.10.2012
(51) Int. Cl.: A61K 8/04, A61K 8/81, A61Q 5/06, A61Q 13/00

(54) **METHOD OF CONTROLLING FRAGRANCE RELEASE ON HAIR**
METHODE ZUR KONTROLLE DER DUFTSTOFFFREISETZUNG AUF HAAREN
MÉTHODE DE CONTROL DE LA LIBERATION DES PARFUMS SUR LES CHEVEUX

(30) Priority: 03.10.2011 US 201161542459 P; 13.12.2011 EP 11193161
(43) Date of publication of application: 02.07.2014
(73) Proprietor: Akzo Nobel Chemicals International B.V., 6824 BM Arnhem (NL)
(72) Inventor: MARTINO, Gary Theodore, Monmouth Junction, New Jersey 08852 (US)
(74) Representative: Akzo Nobel Chemicals IP Group
(86) International application number: PCT/EP2012/069506
(87) International publication number: WO 2013/050398

(56) References cited:
- WO-A1-00/33808
- WO-A1-2005/032503
- DE-A1- 4 034 315
- US-A- 4 196 190
- US-A- 4 923 695
- US-A- 6 110 449
- US-A1- 2002 182 163

## Description

### Field of the invention

The present invention relates to a method of controlling release of a fragrance on hair comprising:
applying a hair styling composition to the hair, wherein said hair styling composition comprises a fragrance and a hair fixative co-polymer comprising at least one first monomer having an acid containing group and at least one second monomer having a hydrophobic group, wherein the hair fixative co-polymer is neutralized from 50% to less than 100%;
drying said hair styling composition to form a film;
and releasing the fragrance from the film upon addition of water.

### Background:

Hair styling fixative polymers have been around for many years to provide the user the ability to place the hair in a given style and to afford a long lasting hold. A key requirement for these fixative polymers is that they are removable by washing the hair using conventional shampoos so that a new style can be readily and easily be created.

Further, the fixative polymer needs to be easily applied to the hair in a uniform and effective manner. This can be achieved by means such as sprays (aerosol or mechanical pump) and with gels. Gels are generally applied to the hair prior to setting, whereas the sprays may be applied to the hair after curlers are used.

During the application of the fixative system to the hair, most consumers prefer the fixative system to have a pleasant fragrance during application. However, the fragrance is typically lost quickly after applying. Deposition of droplets of fragrance (typically as an emulsion) typically interfere with the ability of the fixatives to hold the hair and to resist humidity. Moreover, it is generally desirable to have the hair stay in place even on humid days. One method of addressing these concerns is the addition of surfactants to prepare and stabilize the fragrance emulsion, but this can have the adverse affect of making the fixative more susceptible to moisture and wetting, which in turn decreases the system's ability to resist moisture. WO2005/032503 discloses a controlled delivery system for fragrance comprising a film-forming ingredient consisting of an acrylates/hydroxyacrylates copolymer.

To this end, there still exists a need for providing a long lasting fragrance delivery system for hair styling fixative systems without adversely affecting the hold or humidity resistance.

### Summary of the invention

Disclosed is a hair styling composition comprising at least one volatile compound and a hair fixative co-polymer. The hair fixative co-polymer comprises at least one first monomer having an acid containing group and at least one second monomer having a hydrophobic group.

Also disclosed is a hair styling composition comprising a hair fixative co-polymer and a fragrance. The hair fixative co-polymer comprises a first monomer having an acid containing group, a second monomer containing a hydrophobic group and a third monomer having a hydrophilic group.

Also disclosed is a method for producing a hair styling composition comprising dissolving a hair fixative copolymer in a solvent, neutralizing the solution, and blending a volatile compound into the solution. The co-polymer comprises at least one first monomer having an acid containing group and at least one second monomer having a hydrophobic group.

### Detailed description of the invention

Carboxylic acid-containing hair styling polymers of this invention can be combined with volatile compounds, such as fragrances or perfumes in formulations to provide hair styling products. Typical styling products are applied by either a spray (pump or aerosol) or from a mousse or gel. Each application method has certain unique formulation requirements, such as propellant in the case of an aerosol or gelling agent in the case of mousse or gelled product. Compatibility with the other ingredients is a critical property of the polymer to prevent it from coming out of solution (precipitating or hazing). This can be manipulated by varying the amount and type of other monomers in the styling polymer. For example hydrophobic monomers, such as (meth)acrylates, contribute to the compatibility with propellants and non-aqueous solvents, whereas anionic and cationic monomers will tend to make the polymers more water soluble and provide better compatibility in gel and mousse systems. Examples of (meth)acrylates, include but are not limited to, methylacrylate ethylacrylate, butylacrylate, methylmethacrylate and butylmethacrylate and combinations thereof.

The co-polymers of this invention contain at least two classes of monomers. These two classes include hydrophobic monomers and carboxylic acid monomers. Other monomers can be incorporated into the co-polymer in an amount to provide additional functionality, but are typically incorporated in small amounts, for example generally about 20% or less of the total polymer weight, and in another embodiment about 10% or less and in another embodiment about 5% or less.

The first family of monomers in the co-polymer of this invention are acid-containing monomers. These include any ethylenically unsaturated monomers having a free carboxylic acid group. Some non-limiting examples of these acid-containing monomers are maleic acid, fumaric acid, acrylic acid, methacrylic acid, itoconic acid. In an embodiment of the current invention the co-polymer will contain a mixture of one or more acid-containing monomers.

In an embodiment of the invention, the one or more acid-containing monomers will be present in the co-polymer at a level of about 5 to about 25 weight percent of the total weight of the dry, un-neutralized polymer. In another embodiment, the acid monomers will be present in an amount of about 10 to about 20 weight percent of the dry un-neutralized co-polymer, and in another embodiment about 12 to about 20 weight percent. In another embodiment, the polymer will have an acid number of about 1.5 to about 3.2, as determined by titration with 0.1 N KOH, where the acid number is reported as the number of milligrams of KOH needed to neutralize one gram of polymer. In yet another embodiment the co-polymer will have an acid number of from about 1.8 to about 2.6 as determined by titration with 0.1 N KOH.

The second family of monomers contained in the co-polymer of this invention are hydrophobic monomers. This class of monomers are ethylenically unsaturated monomers and are uncharged (i.e. nonionic) and generally have an alkyl group of from 1 to about 12 carbon atoms, and in another embodiment from about 1 to about 8 carbon atoms. The monomers can be based on acrylic type monomers, such as methylmethacrylate or ethylacrylate. These hydrophobic monomers can also be based on acrylamide type monomers such as t-octylacrylamide, butylacrylamide, and methylacylamide. In an embodiment of the current invention the co-polymer will contain a mixture of one or more hydrophobic monomers.

In an embodiment of the invention, the one or more hydrophobic monomers will be present in the co-polymer from about 65 to about 85 weight percent of the dry un-neutralized co-polymer and in another embodiment 68 to 83 weight percent. In yet another embodiment the hydrophobic monomer will be present in co-polymer from about 70 to about 80 weight percent of the dry un-neutralized polymer weight. In another embodiment of the invention the hydrophobic monomer will be chosen from the group consisting of methylmethacrylate, ethylmethacrylate, t-octylacrylamide, ethylacrylamide, t-butylmethacrylate or mixtures thereof.

A third class of optional monomers that may be present in the co-polymer of this invention are hydrophilic monomers. This class of ethylenically unsaturated monomers generally contains monomers with hydroxyl groups or amine groups. Some examples of these monomers are hydroxypropylmethacrylate, t-butylaminoethylmethacrylate. In an embodiment of the current invention the co-polymer will contain a mixture of one or more hydrophilic monomers. In an embodiment of the invention, the one or more hydrophilic monomers are present in an amount of about 1 to about 25 weight percent of the dry un-neutralized co-polymer and in another embodiment of from about 5 to about 15 weight percent of the dry un-neutralized co-polymer.
Some non-limiting examples of the polymers of this invention are: octylacrylamide/acrylates/butylaminoethyl methacrylate copolymer (e.g. Amphomer® resin), Acrylates Copolymer (e.g., Luvimer® Polymer is a terpolymer of t-butyl acrylate/ethyl acrylate and methacrylic acid), a copolymer of VA/Crotonates/Vinyl Neodecanoate Copolymer such as Resyn® 28-2913(a terpolymer of vinyl acetate, crotonic acid and vinyl neodecanoate) and other commercially available polymers of this type. Amphomer® and Resyn® are available from (Akzo Nobel Personal Care LLC, Bridgewater, New Jersey 08559) and Luvimer® polymers are available from BASF Aktiengesellschaft, 67056 Ludwigshafen, Germany).

Optional polymers, individually or in combination, can also be added to the hair fixative formulation in addition to the polymer of this invention. Some non-limiting examples are Polyquaternium-11, such as Gafquat® 734 or Gafquat® 755N; a hydrophilic Polyether Urethane such as Polyurethane Resin 142-89; a Polyquaternium 4 such as Celquat® L-200 or Celquat® H-100; a polyvinylpyrrollidine such as PVP K-60, K-90, K-120 (PVP); a copolymer of PVP/VA such as 335, 535, 735, 630;; a copolymer of Acrylate/Acrylamide Copolymer such as Ultrahold; a PVP/Dimethylaminoethyl methacrylate copolymer such as Copolymer 845; and a PVP/VA Copolymer such as E-635, Gantrez® 425, 335, and 215 and a copolymer of PVM/MA Decadiene Crosspolymer such as LoVocryl®.

Another desirable property of the styling polymer is shampoo removability. Residual polymer left on the hair after a single wash cycle will lead to unpleasant build-up over time and a sticky feel to the touch. To accomplish this property the amount of the anionic monomer is controlled with respect to the other monomers and then neutralized after polymerization to generate the carboxylic acid salt(s). Typical neutralizing agents are hydroxides of alkaline and alkaline earth metals, organic amines, carbonate and bicarbonate salts. In an embodiment of this invention the neutralizing agent is chosen from the group consisting of triethanol amine (TEA), 2-amino-2-methyl-1-propanol (AMP), sodium hydroxide, potassium hydroxide and sodium carbonate and combinations thereof.

The polymers of the instant invention have at least some of the carboxylic acid monomer residues neutralized to provide the appropriate amount of water sensitivity. If the water sensitivity is too high, the polymer film will release the fragrance too rapidly and not provide the long lasting scent. If the polymer film is too insensitive to water, the film will form a semi-permanent entrapment and will not release the fragrance in a high enough concentration to be detected by the user. In an embodiment of this invention the carboxylic acid groups of the co-polymer (acid containing monomers described above) will be neutralized from 50% to less than 100%. In another embodiment the acid groups will be neutralized from 50 to 95% and in another embodiment the neutralization of the co-polymer is from 75 to 95%. In yet another embodiment the acid groups will be neutralized from 75 to 90%, and in another embodiment from 75% to 85%. In still another embodiment the acid groups will be neutralized from 85 to 95%.

The volatile compounds suitable for use in the invention can be derived from synthetic or all natural sources. These may include essential oils and blends of synthetic and essential oils. In an embodiment, the volatile compounds may include fragrance extracts such as would be found in common plants and herbs such as mint, clove, lavender and tea tree oil. Also included within the scope of volatile compounds suitable for use in this invention are complex blends of extracts (such as would be found in many proprietary fragrances), as well as herbal extracts such as sterols, triterpenes, flavonoids, coumarins, non-glycosidic diterpenes (sterebins) spathulenol, decanoic acid, 8,11,14-ecosatrienoic acid, 2-methyloctadecane, pentacosane, octacosane, stigmasterol, bsitosterol, a- and b-amyrine, lupeol, b-amyrin acetate, and pentacyclic triterpene. Food extracts are also included under this definition of fragrance. Non- limiting examples of these would be vanilla, chocolate, strawberry, and mint. In an embodiment, the volatile compound is a volatile oil. In another embodiment, the volatile compound can be a mixture volatile compounds.

In another embodiment of the invention, non-volatile compounds such as certain oils, may also optionally be included and can include sunscreen actives such as such as a p-methoxycinnamate or an aminobenzoate (UVB blocker) or benzone or an anthranilate (UVA blocker). Non-volatile compounds, such as medicaments, moisturizers, anti-itch or anti-dandruff ingredients and mixtures thereof can also be applied.

The hair styling composition of this invention will contain an effective amount of the fixative co-polymer. In an embodiment of this invention the fixative co-polymer will be present from about 0.1 to about 5 weight percent of the total weight of the formulation. In another embodiment the fixative co-polymer can be present at from about 1 to about 3% of the total weight of the formation.

Additionally the hair styling formulations of the invention may contain a solvent for applying the fixative formulation. Solvents, as defined herein, include ethanol, isopropanol, chlorinated or fluorinated hydrocarbons, water or mixtures thereof. In an embodiment of this invention, the formulation will contain no more than about 85% of volatile organic compounds (VOCs, aka-solvents or propellants), with the remainder of the solvent being water. In another embodiment, the formulation will contain no more than about 55% VOCs.

Disclosed is a method for producing a hair styling composition comprising dissolving a hair fixative copolymer in the solvent, neutralizing the resultant solution and blending a volatile compound, such as a fragrance, into the solution.

In an embodiment of the invention, the co-polymer is used in an aerosol formulation, wherein one or more propellants is present. In an embodiment, propellants suitable for use in the invention include, but are not limited to, hydrocarbons, fluorinated hydrocarbons, chlorinated hydrocarbons, compressed gas, dimethyl ether and compressed volatile liquids. A skilled artisan will recognize that these propellants will generally add to the overall VOC of the formulation and that the polymer system must be compatible with the propellant to avoid problems (nozzle clogging, spitting, poor spray pattern, etc.) during the atomization and application to the hair.

Not wishing to be bound by theory, it is believed that the co-polymers of the instant invention entrap the fragrance within the film and slowly allow the fragrance to diffuse out of the film particularly on contact with moisture. The fixative co-polymer systems of this invention, when properly neutralized and formulated, will give the wearer a fresh burst of fragrance when the hair is moistened, such as from sweat, rain or high humidity.

The invention thus provides a method of providing controlled release of a fragrance on hair comprising applying the hair styling composition to the hair, drying the hair styling composition to form a film and releasing the fragrance from the resultant film upon addition of water. In an embodiment, the fragrance is encapsulated in the film.

It has been found that it is possible to vary the ratio of the monomers and the level of neutralization of the fixative polymer to alter the amount of release of the fragrance for long or short duration once the hair has been contacted with the moisture (i.e. water). For example, the sweat from a cardio workout might trigger a rapid release of the fragrance, whereas high humidity may trigger a slow and longer lasting release; all the while providing the hair with a consistent style, look and feel. In an embodiment of the invention, the volatile compound, such as a fragrance, is released upon addition of moisture. The amount of moisture may be about 20% or greater based on the weight of the dry styled hair. In an embodiment of this invention the moisture will be 40% or greater based on the weight of the dry styled hair. In another embodiment of this invention the moisture will be about 60% or greater based on the weight of the dry styled hair. In yet another embodiment of this invention the moisture will be about 80% or greater based on the weight of the dry styled hair.

Additionally, a variety of one or more adjunct or ancillary ingredients may be added to the hair fixative composition to provide functions other than fixative properties.

Examples of these ancillary ingredients are preservatives, colorants, viscosity modifiers, vitamins, and the like.

The following examples are intended to exemplify the present invention but are not intended to limit the scope of the invention in any way. The breadth and scope of the invention are to be limited solely by the claims appended hereto.

### Examples:

Various polymers were tested to compare their respective fragrance retentions at 0.5, 3, 6 hours after application. Subjective evaluations of the dry hair were compared

### Preparation of hair fixative formulations:

Sample 1 was prepared by mixing 0.3 g of fragrance (Pure Seduction) with 59.7 grams of ethanol in a 150 mL beaker until uniform.

Sample 2 was prepared by adding 56.7 grams of ethanol to a 150 mL beaker and stirred with a magnetic stirring bar. The Amphomer® co-polymer (3.0 grams) was then added to the by slowly sprinkling the polymer onto the surface of the ethanol over about 2 minutes. The solution was stirrer until clear and homogeneous. A total of 0.3 grams of fragrance (Pure Seduction) was added drop wise to the stirring solution.

Sample 3 was prepared adding 56.15 grams of ethanol to a 150 mL beaker and stirred with a magnetic stirring bar. The Amphomer® copolymer (3.0 grams) was then added to the stirring ethanol by slowly sprinkling the polymer onto the surface of the ethanol over about 2 minutes. The solution was stirrer until clear and homogeneous. The Amphomer® co-polymer is then neutralized with 0.55 grams of 2-amino-2-methyl-1-propanol (AMP) by adding the AMP drop wise over about 2-3 minutes to the solution. Once the AMP addition is complete a total of 0.3 grams of fragrance (Pure Seduction) was added drop wise to the stirring solution.

Sample 4 was prepared using the same procedure as sample 2 (above) except that PVP-K90 was used in place of the Amphomer® co-polymer.

The samples from above were then charged into an aerosol can and 40 grams of dimethyl ether (DME) is added to the can under pressure and the can is sealed with the spray actuator.
The following samples were evaluated.
Sample 1 - Ethanol/Fragrance/DME (control)
Sample 2 - Ethanol/Fragrance/DME/Amphomer (unneutralized)
Sample 3 - Ethanol/Fragrance/DME/Amphomer (99% AMP neutralized)
Sample 4 - Ethanol/Fragrance/DME/PVP_K-90

Each of the sample had 0.5% of Pure Seduction (fragrance available from the website fragrances and flavors.com added to them prior to application to the hair.

The hair samples were prepared by first washing the hair swatch with non-scented soap and were then rinsed with DI water. The wet hair swatch was then treated with three (3) applications of 0.16 mL of sample applied from a pump spray actuator. The samples were allowed to dry in an oven for 5 minutes at 40°C and then subjected to panel evaluation

Different samples of hair spray were compared to a control lot using the following tests and formula:
- Subjective evaluation of the dry hair at 0.5, 3, 6 hours
- Add Moistening step and re-evaluate the subjective test

**Formula:**

| | |
|---|---|
| DME | 40% |
| Polymer | 3% |
| Fragrance | 0.30% |

### Observation:

There was a significant difference between the Samples 3 and #4. Regardless of what size actuator was used, the sample that included PVP K-90 sprayed poorly and did not atomize well. This resulted in non-uniform application and poor aesthetics.

### Subjective Performance Evaluations:

Subjective evaluation was then completed comparing all three samples, i.e. Samples 2-4 to the control, i.e. Sample 1, after 0.5, 3, and 6 hours. Panelists were required to smell the hair swatches and indicate which one smelled stronger. After 6 hrs, moisture was added to the each of the Samples by simply misting the treated hair swatch from a spray bottle to see if there were any detectable differences.

A total of eight (8) panelists were allowed to smell the treated hair swatch. The number reported in table 1 indicates where the sample had more (+) or less (-) smell and the number of panelists that detected the difference (0/8, meaning that none of the panelist detected a positive level compared to the control; (8/8, indicates that all of the panelists detected a positive difference compared to the control)

As illustrated in the in the chart below Sample #2 and Sample#3 found to be inferior after 0.5, 3, 6 hrs when compare to the control, Sample 1. Whereas Sample # 3 was found to be superior after adding the moisture (indicating fragrance was released), Sample#4 was found to be superior after 0.5, 3, 6 hrs, but did not give a boost in fragrance release when the moisture was added.

**Table 1 - Panel evaluation**

| | Sample #1 | Sample #2 | Sample #3 | Sample #4 |
|---|---|---|---|---|
| 30 min. | = | - | - | + |
| | | (0/8) | (0/8) | (8/8) |
| 3 hrs | = | - | - | + |
| | | (0/8) | (0/8) | (7/8) |
| 6 hrs | = | - | - | + |
| | | (0/8) | (0/8) | (7/8) |
| Moisture added | = | - | + | - |
| | | (1/8) | (7/8) | (2/8) |

Based on the result above, Sample #3 gave an unexpected increase in fragrance release on addition of moisture over the un-neutralized Sample (#2) and the water soluble polymer Sample (#4). It should be noted that the fragrance release of the control Sample #1 and Sample #4 quickly diminished over the first 6 hours to a rather low level as compared to Sample #3, which had very little release until the moisture was added.

While particular embodiments of the present invention have been illustrated and described herein, the invention is not intended to be limited to the details shown. Rather, various modifications may be made in the details within the range and scope of equivalents of the claims.

## Claims

1. A method of controlling release of a fragrance on hair comprising:
applying a hair styling composition to the hair, wherein said hair styling composition comprises:
a fragrance; and
a hair fixative co-polymer comprising at least one first monomer having an acid containing group and at least one second monomer having a hydrophobic group, wherein the hair fixative co-polymer is neutralized from 50% to less than 100%;
drying said hair styling composition to form a film; and
releasing the fragrance from the film upon addition of water.

2. The method of claim 1, wherein the fragrance is encapsulated in the film.

3. The method of claims 1 or 2 wherein the hair fixative co-polymer is neutralized from 50% to 95%.

4. The method of claims 1-3 wherein the hair fixative co-polymer is present in the hair styling composition in an amount from 0.1 to 5 weight percent based on total weight of the hair styling composition.

5. The method of claims 1-4 wherein hair fixative co-polymer is neutralized with hydroxides of alkaline metals, hydroxides of alkaline earth metals, organic amines, carbonates or bicarbonate salts.

6. The method of claims 1-5 wherein the hair styling composition further comprises a propellant selected from the group consisting of hydrocarbons, fluorinated hydrocarbons, chlorinated hydrocarbons, compressed gas, dimethyl ether and compressed volatile liquids.

7. The method of claims 1-6 wherein the hair styling composition further comprises a solvent selected from the group consisting of ethanol; isopropanol, chlorinated hydrocarbons, fluorinated hydrocarbons, water and mixtures thereof.

8. The method of claim 7, wherein the hair fixative co-polymer and the fragrance are present in the solvent.

9. The method of claims 1-8 wherein the at least one first monomer is selected from the group consisting of maleic acid, fumaric acid, acrylic acid, methacrylic acid and itaconic acid and mixtures thereof.

10. The method of claims 1-9 wherein the at least one first monomer is present in the hair fixative co-polymer in an amount of 5 to 25 weight percent of the total weight of the dry, un-neutralized co-polymer.

11. The method of claims 1-10 wherein the at least one second monomer is an uncharged ethylenically unsaturated monomer having a hydrophobic alkyl group of from 1 to 12 carbon atoms.

12. The method of claims 1-11 wherein the at least one second monomer is selected from the group consisting of t-octylacrylamide, butylacrylamide, methylacrylamide, methylmethacrylate, ethylacrylate and mixtures thereof.

13. The method of claims 1-12 wherein the at least one second monomer is present in the hair fixative co-polymer in an amount of 68 to 83 weight percent of the dry, un-neutralized co-polymer.

14. The method of claims 1-13 wherein a hair fixative co-polymer further comprises at least one third monomer wherein said at least one third monomer is an ethylenically unsaturated monomer having hydroxyl groups or amine groups.

15. The method of claims 1-14 wherein the at least one third monomer is hydroxypropylmethacrylate, butylarninoethylmethacrylate or mixtures thereof.

## Patentansprüche

1. Verfahren zum Steuern der Freigabe eines Duftstoffes auf Haaren, umfassend:
Auftragen einer Haarstyling-Zusammensetzung auf das Haar, wobei die Haarstyling-Zusammensetzung Folgendes umfasst:
einen Duftstoff und
ein Haarfestiger-Copolymer, umfassend wenigstens ein erstes Monomer mit einer säurehaltigen Gruppe und wenigstens ein zweites Monomer mit einer hydrophoben Gruppe, wobei das Haarfestiger-Copolymer von 50% auf weniger als 100% neutralisiert ist;
Trocknen der Haarstyling-Zusammensetzung, um einen Film zu bilden, und
Freigeben des Duftstoffes aus dem Film bei Zugabe von Wasser.

2. Verfahren nach Anspruch 1, wobei der Duftstoff in dem Film eingekapselt ist.

3. Verfahren nach Anspruch 1 oder 2, wobei das Haarfestiger-Copolymer von 50% auf 95% neutralisiert ist.

4. Verfahren nach Anspruch 1-3,wobei das Haarfestiger-Copolymer in der Haarstyling-Zusammensetzung in einer Menge von 0,1 bis 5 Gew.-%, basierend auf dem Gesamtgewicht der Haarstyling-Zusammensetzung, vorhanden ist.

5. Verfahren nach Anspruch 1-4, wobei das Haarfestiger-Copolymer mit Hydroxiden von Alkalimetallen, Hydroxiden von Erdalkalimetallen, organischen Aminen, Carbonaten oder Bicarbonatsalzen neutralisiert ist.

6. Verfahren nach Anspruch 1-5, wobei die Haarstyling-Zusammensetzung weiterhin ein Treibmittel umfasst, ausgewählt aus der Gruppe, bestehend aus Kohlenwasserstoffen, fluorierten Kohlenwasserstoffen, chlorierten Kohlenwasserstoffen, Druckgas, Dimethylether und komprimierten, flüchtigen Flüssigkeiten.

7. Verfahren nach Anspruch 1-6, wobei die Haarstyling-Zusammensetzung weiterhin ein Lösungsmittel umfasst, ausgewählt aus der Gruppe, bestehend aus Ethanol, Isopropanol, chlorierten Kohlenwasserstoffen, fluorierten Kohlenwasserstoffen, Wasser und Mischungen daraus.

8. Verfahren nach Anspruch 7, wobei das Haarfestiger-Copolymer und der Duftstoff in dem Lösungsmittel vorhanden sind.

9. Verfahren nach Anspruch 1-8, wobei das wenigstens eine erste Monomer ausgewählt ist aus der Gruppe, bestehend aus Maleinsäure, Fumarsäure, Acrylsäure, Methacrylsäure und Itaconsäure und Mischungen daraus.

10. Verfahren nach Anspruch 1-9, wobei das wenigstens eine erste Monomer in dem Haarfestiger-Copolymer in einer Menge von 5 bis 25 Gew.-% des Gesamtgewichts des trockenen, unneutralisierten Copolymers vorhanden ist.

11. Verfahren nach Anspruch 1-10, wobei das wenigstens eine zweite Monomer ein ungeladenes, ethylenisch ungesättigtes Monomer mit einer hydrophoben Alkylgruppe von 1 bis 12 Kohlenstoffatomen ist.

12. Verfahren nach Anspruch 1-11, wobei das wenigstens eine zweite Monomer ausgewählt ist aus der Gruppe, bestehend aus t-Octylacrylamid, Butylacrylamid, Methylacrylamid, Methylmethacrylat, Ethylacrylat und Mischungen daraus.

13. Verfahren nach Anspruch 1-12, wobei das wenigstens eine zweite Monomer in dem Haarfestiger-Copolymer in einer Menge von 68 bis 83 Gew.-% des trockenen, unneutralisierten Copolymers vorhanden ist.

14. Verfahren nach Anspruch 1-13, wobei ein Haarfestiger-Copolymer weiterhin wenigstens ein drittes Monomer umfasst, wobei das wenigstens eine dritte Monomer ein ethylenisch ungesättigtes Monomer mit Hydroxylgruppen oder Amingruppen ist.

15. Verfahren nach Anspruch 1-14, wobei das wenigstens eine dritte Monomer Hydroxypropylmethacrylat, Butylaminoethylmethacrylat oder Mischungen daraus ist.

## Revendications

1. Procédé de commande de la libération d'un parfum sur les cheveux comprenant le fait :
d'appliquer une composition de coiffage sur les cheveux, où ladite composition de coiffage comprend :
un parfum ; et
un copolymère fixateur pour cheveux comprenant au moins un premier monomère ayant un groupe contenant un acide et au moins un deuxième monomère ayant un groupe hydrophobe, où le copolymère fixateur pour cheveux est neutralisé de 50% à moins de 100% ;
de sécher ladite composition de coiffage pour former un film ; et
de libérer le parfum du film lors de l'ajout de l'eau.

2. Procédé de la revendication 1, dans lequel le parfum est encapsulé dans le film.

3. Procédé da la revendication 1 ou 2, dans lequel le copolymère fixateur pour cheveux est neutralisé de 50% à 95%.

4. Procédé des revendications 1 à 3, dans lequel le copolymère fixateur pour cheveux est présent dans la composition de coiffage en une quantité allant de 0,1 à 5 pour cent en poids par rapport au poids total de la composition de coiffage.

5. Procédé des revendications 1 à 4, dans lequel le copolymère fixateur pour cheveux est neutralisé par des hydroxydes de métaux alcalins, des hydroxydes de métaux alcalino-terreux, des amines organiques, des carbonates ou des sels de bicarbonate.

6. Procédé des revendications 1 à 5, dans lequel la composition de coiffage comprend en outre un agent propulseur choisi dans le groupe constitué par des hydrocarbures, des hydrocarbures fluorés, des hydrocarbures chlorés, du gaz comprimé, de l'éther diméthylique et des liquides volatils comprimés.

7. Procédé des revendications 1 à 6, dans lequel la composition de coiffage comprend en outre un solvant choisi dans le groupe constitué par l'éthanol, l'isopropanol, des hydrocarbures chlorés, des hydrocarbures fluorés, de l'eau et des mélanges de ceux-ci.

8. Procédé de la revendication 7, dans lequel le copolymère fixateur pour cheveux et le parfum sont présents dans le solvant.

9. Procédé des revendications 1 à 8, dans lequel l'au moins un premier monomère est choisi dans le groupe constitué par l'acide maléique, l'acide fumarique, l'acide acrylique, l'acide méthacrylique et l'acide itaconique et des mélanges de ceux-ci.

10. Procédé des revendications 1 à 9, dans lequel l'au moins un premier monomère est présent dans le copolymère fixateur pour cheveux en une quantité allant de 5 à 25 pour cent en poids du poids total du copolymère sec non neutralisé.

11. Procédé des revendications 1 à 10, dans lequel l'au moins un deuxième monomère est un monomère à insaturation éthylénique non chargé comportant un groupe alkyle hydrophobe ayant 1 à 12 atome(s) de carbone.

12. Procédé des revendications 1 à 11, dans lequel l'au moins un deuxième monomère est choisi dans le groupe constitué par le t-octylacrylamide, le butylacrylamide, le méthylacrylamide, le méthacrylate de méthyle, l'acrylate d'éthyle et des mélanges de ceux-ci.

13. Procédé des revendications 1 à 12, dans lequel l'au moins un deuxième monomère est présent dans le copolymère fixateur pour cheveux en une quantité allant de 68 à 83 pour cent en poids du copolymère sec non neutralisé.

14. Procédé des revendications 1 à 13, dans lequel un copolymère fixateur pour cheveux comprend en outre au moins un troisième monomère, où ledit au moins un troisième monomère est un monomère à insaturation éthylénique ayant des groupes hydroxyle ou des groupes amine.

15. Procédé des revendications 1 à 14, dans lequel l'au moins un troisième monomère est le méthacrylate d'hydroxypropyle, le méthacrylate de butylaminoéthyle ou des mélanges de ceux-ci.
